Europäisches Patentamt

European Patent Office

Office européen des brevets

(19)

(11) **EP 0 806 978 B1**

(12) **EUROPÄISCHE PATENTSCHRIFT**

(45) Veröffentlichungstag und Bekanntmachung des Hinweises auf die Patenterteilung:
**15.03.2000 Patentblatt 2000/11**

(51) Int Cl.$^7$: **B01D 19/04**, C07C 59/125

(21) Anmeldenummer: **96901754.0**

(86) Internationale Anmeldenummer:
**PCT/EP96/00257**

(22) Anmeldetag: **23.01.1996**

(87) Internationale Veröffentlichungsnummer:
**WO 96/23568 (08.08.1996 Gazette 1996/36)**

(54) **VERWENDUNG VON ALKOXYLIERUNGSPRODUKTEN EPOXIDIERTER FETTSTOFFE ALS ENTSCHÄUMER**

USE OF ALKOXYLATION PRODUCTS OF EPOXIDIZED FATS AS ANTIFOAMING AGENTS

EMPLOI DE PRODUITS D'ALCOXYLATION DE MATIERES GRASSES EPOXYDEES COMME AGENTS ANTIMOUSSE

(84) Benannte Vertragsstaaten:
**AT BE CH DE DK ES FR GB GR IE IT LI NL PT SE**

(30) Priorität: **01.02.1995 DE 19503062**

(43) Veröffentlichungstag der Anmeldung:
**19.11.1997 Patentblatt 1997/47**

(73) Patentinhaber: **Cognis Deutschland GmbH
40589 Düsseldorf (DE)**

(72) Erfinder:
• **WOLLENWEBER, Horst-Werner
  D-40597 Düsseldorf (DE)**
• **HORNFECK, Klaus
  D-40822 Mettmann (DE)**
• **KAPS, Dieter
  D-51375 Leverkusen (DE)**
• **NELLESSEN, Bernhard
  D-41564 Kaarst (DE)**
• **KÖSTER, Rita
  D-40479 Düsseldorf (DE)**
• **DREES, Wolfgang
  D-40593 Düsseldorf (DE)**
• **DAUTE, Peter
  D-27616 Beverstedt (DE)**

(56) Entgegenhaltungen:
• **DATABASE WPI Section Ch, Week 9104 Derwent Publications Ltd., London, GB; Class A25, AN 91-023236 & DE,A,39 23 394 ( HENKEL KGAA) , 17.Januar 1991 in der Anmeldung erwähnt**
• **DATABASE WPI Section Ch, Week 9350 Derwent Publications Ltd., London, GB; Class A97, AN 93-405795 & WO,A,93 24603 ( UNILEVER PLC) , 9.Dezember 1993**
• **DATABASE WPI Section Ch, Week 9104 Derwent Publications Ltd., London, GB; Class A97, AN 91-023235 & DE,A,39 23 393 ( HENKEL KGAA) , 17.Januar 1991 in der Anmeldung erwähnt**

**Beschreibung**

**Gebiet der Erfindung**

Die Erfindung betrifft die Verwendung von Alkoxylierungsprodukten epoxidierter Fettstoffe als Entschäumer bei der Papierherstellung, der Lebensmittelherstellung, wie der Zucker- oder Stärkegewinnung, der Fermentation, der Textilfärbung und in Lacken.

**Stand der Technik**

Für eine Vielzahl technischer Prozesse stellt die mit der Anwesenheit bzw. mit dem Einsatz von grenzflächenaktiven Substanzen verbundene Schaumentwicklung ein ernsthaftes Problem dar.

In der Papierfabrikation können aufgrund des im Wasserkreislauf eingetragenen Luftgehaltes Schäume gebildet werden, die zu Störungen führen. Beispielsweise können Schaumflecken auf dem Papier auftreten, wenn Schaum mit flotiertem Schmutz bei der Blattbildung auf die Papierbahn gelangt. Durch die zunehmend schneller laufenden Maschinen steigt insgesamt die Gefahr der Untermischung von Luft in die Fasersuspension, was zu einer Störung des Entwässerungsvorgangs des Papierstoffs auf der Papiermaschine und letztendlich zu ungleichmäßigen Strukturen innerhalb des Papierblattes führen kann. Diese prinzipiell bekannten Nachteile werden durch die neuen Papiermaschinen mit den geschlosseneren Wasserkreisläufen noch verstärkt, da sich schaumbildende und schaumstabilisierende Stoffe in den geschlossenen Systemen anreichern. Wie die obigen Ausführungen zeigen, besteht in der Technik ein großer Bedarf sowohl an Entschäumern, die in der Lage sein sollen, entstandenen Schaum zu verringern, als auch an Schauminhibitoren, die das Entstehen von Schaum unterdrücken sollen. Von den Schauminhibitoren wird erwartet, daß sie in geringen Zusatzmengen spontan, aber auch langanhaltend wirksam sind.

Bei der industriellen Herstellung oder Verarbeitung von Nahrungsmitteln kommt der Bekämpfung und Verhütung von auftretendem Schaum beträchtliche Bedeutung zu. So können beispielsweise bei der industriellen Verarbeitung zuckerhaltiger Pflanzensäfte, wie sie im großen Maßstab bei der Gewinnung von Zucker aus Rüben erfolgt, besondere Schwierigkeiten durch übermäßige Schaumentwicklung in der Saftgewinnung, -reinigung und in den Verdampfern auftreten. Die in der Zuckerindustrie einzusetzenden Schaumbekämpfungsmittel müssen selbstverständlich physiologisch unbedenklich sein. Dasselbe gilt für Schaumbekämpfungsmittel, die bei der Herstellung von Kartoffelfertigprodukten wie Kartoffelchips oder Pommes Frites oder aber auch bei der Erzeugung von Backhefe unter Verwendung von Melasse eingesetzt werden. Zusätzlich müssen die Schaumbekämpfungsmittel in der kartoffelverarbeitenden Industrie dazu in der Lage sein, den schwer zu bekämpfenden Stärkeschaum zu regulieren. Insgesamt erwartet der Praktiker zudem stets Schaumbekämpfungsmittel, die in kleinen Anwendungsmengen hohe Spontan- und Langzeitwirkung haben.

Für die Entschäumung in der Zucker- und Hefeindustrie sind seit langem Fette und Öle wie Rüb-, Erdnuß-, Olivenöle sowie Wollfett in Gebrauch. Auch synthetische Ester wie Fettsäuremono- und -diglyceride, Fettsäurepolyglykolester und synthetische Alkohole wie Polyalkylenglykole und Alkylenoxidaddukte an Fettalkohole sind für diesen Zweck vorgeschlagen worden. Obgleich mit diesen Verbindungen eine gewisse Schaumunterdrückung erreicht werden kann, ist jedoch häufig die notwendige Einsatzmenge zu hoch, beziehungsweise die Wirksamkeit nur bei bestimmten Temperaturen gegeben.

Bei Lacken und Farben, insbesondere auf Basis von Wasser als alleinigem oder überwiegendem Lösungsmittel, kann durch Homogenisieren der Lack- beziehungsweise Farbbestandteile Luft eingerührt werden. Dies ist besonders nachteilig, da entweder sehr lange vom Anwender gewartet werden muß, bis diese Luftbläschen zerplatzt sind, bevor er mit dem Lack- beziehungsweise Farbaufstrich beginnen kann oder der Lack- beziehungsweise Farbaufstrich zeigt Bläschen. Derartige Aufstriche mit Oberflächenstörungen sind nicht nur optisch unschön, sondern auch weniger haltbar, da die Bläschen platzen können und dabei Löcher oder Schwachstellen im Film hinterlassen.

Zur Behebung der Schaumprobleme sind bereits zahlreiche Verbindungsklassen vorgeschlagen worden.

In der DE-A-40 38 608 werden Ringöffnungsprodukte epoxidierter Triglyceride als Entschäumer in der Nahrungsmittel-Industrie vorgeschlagen.

Die DE-A-38 15 826 lehrt, Epoxystearinsäuremethylester mit Carbonsäuren unter Ringöffnung abreagieren zu lassen und nachfolgend die entstandene Hydroxylgruppe mit Essigsäureanhydrid zu acylieren und die Verwendung dieser Verbindung als Entschäumer.

Die DE-A-39 23 394 beansprucht ein Verfahren zur Alkoxylierung ring-geöffneter epoxidierter Fettsäureester, die DE-A1 39 23 393 die Verwendung dieser Verbindungen zur Unterstützung der Entfärbung von Altpapier.

Die deutsche Patentanmeldung DE-A-4 331 229 beschreibt die Verwendung von Blockcopolymeren aus mit Alkoholen ringgeöffneten epoxidierten ungesättigten Carbonsäuren und Polyethylenglykol als Entschäumer.

Die Aufgabe bestand in der Bereitstellung eines möglichst universell in zum Schäumen neigenden wäßrigen Systemen einsetzbaren Schaumregulators, der in geringen Mengen eine gute Spontanwirkung zeigt und diese Wirkung möglichst lange behält. Zudem sollte er so maßgeschneidert werden können, daß er schon bei niedrigen Temperaturen wirksam ist und dennoch über einen weiten Temperaturbereich seine Wirkung entfaltet. Weiterhin soll sowohl der Schaumregulator im Gemisch mit sonstigen üblichen Bestandteilen der zu entschäumenden wäßrigen Systeme lager- und wirkungsstabil bleiben und keine nachteiligen Auswirkungen auf das Behandlungsgut und die Umwelt ausüben. Zum Einsatz in der Farben- und Lackindustrie sollten die Schaumregulatoren nicht nur eine gute Entschäumerwirkung haben, sondern auch einen homogenen Lack- oder Farbanstrich ermöglichen. Dies gilt insbesondere auch für das Papiercoating, für das ein homogener gleichmäßiger Farbstrich möglichst ohne Oberflächenstörung notwendig ist.

Überraschenderweise wurde nun gefunden, daß diese Aufgabe durch die Verwendung von Ethoxylierungsprodukten von mit Fettsäuren ringgeöffneten epoxidierten Triglyceriden als Entschäumer gelöst werden kann.

## Beschreibung der Erfindung

Gegenstand der Erfindung ist die Verwendung von Alkoxylierungsprodukten von mit Nucleophilen ringgeöffneten epoxidierten Fettstoffen als Entschäumer.

Unter epoxidierten Fettstoffen sind olefinisch ungesättigte Fettsäure-Ester oder Fettalkohole oder deren Ester zu verstehen, deren Doppelbindungen zu Epoxidgruppen oder Oxiranringen epoxidiert wurden. Wenn man diese Epoxidgruppen mit einem Nucleophil H-X abreagieren läßt, wird X unter Ausbildung einer OH-Gruppe an den Epoxidring addiert. Diese OH-Gruppe kann weiter mit Alkylenoxiden umgesetzt werden.

Geeignete Alkylenoxide sind z.B. Ethylenoxid, Propylenoxid und Butylenoxid. Bevorzugt erfolgt die Umsetzung der gebildeten OH-Gruppen mit Ethylen- und/oder Propylenoxid.

Beispiele epoxidierter Fettstoffen sind epoxidierte Triglyceride, epoxidierte Fettsäureester monofunktioneller Alkohole und epoxidierte Fettalkohole oder deren Ester, bevorzugt mit monofunktionellen Carbonsäuren.

Unter olefinisch ungesättigten Fettsäuren sind Carbonsäuren mit 8 bis 22 C-Atomen und 1 bis 5 Doppelbindungen zu verstehen. Die olefinisch ungesättigten Fettalkohole leiten sich durch Reduktion der Carboxylgruppe von den olefinisch ungesättigten Fettsäuren ab.

Entsprechend ihrer Gewinnung aus natürlichen Fetten und Ölen stellen Fettsäuren überwiegend Gemische verschiedener Homologe dar. Die epoxidierten Fettstoffe können deshalb auch gesättigte Fettsäuren und Fettalkohole enthalten, und zwar bevorzugt nicht mehr als 35 Gew.%, bezogen auf die Summe aus gesättigten und ungesättigten Fettstoffen.

Geeignete epoxidierte Triglyceride, also epoxidierte Fettsäureglycerinester, kann man aus einer Vielzahl von Triglyceriden pflanzlichen oder tierischen Ursprungs gewinnen. So sind beispielsweise epoxidierte Triglyceride geeignet, die 2 bis 10 Gewichtsprozent Epoxidsauerstoff aufweisen. Derartige Produkte sind durch Epoxidation der Doppelbindungen aus einer Reihe von Fetten und Ölen herstellbar, z.B. Rindertalg, Palmöl, Erdnußöl, Rüböl, Korianderöl, Olivenöl, Fischöl, Baumwollsaatöl, Sojaöl, Sonnenblumenöl und Leinöl. Besonders bevorzugte epoxidierte Triglyceride sind epoxidiertes Sojaöl und epoxidiertes Leinöl.

Geeignet sind auch epoxidierte Fettsäureester von Alkoholen mit 1 bis 4 C-Atomen, also epoxidierter Fettsäuremethyl-, -ethyl-, -propyl- oder -butylester.

Typische Beispiele sind die Epoxide von Palmitoleylsäuremethylester, Ölsäuremethylester, Elaidinsäuremethylester, Petroselinsäuremethylester, Linolsäuremethylester, Erucasäuremethylester, Ölsäurebutylester oder Ölsäure-i-butylester.

Auch epoxidierte Fettalkohole mit 12 bis 22 C-Atomen oder deren Ester mit bevorzugt monofunktionellen Carbonsäuren, z.B. epoxidierter Essigsäureoleylester, Ölsäureoleylester oder Erucasäureoleylester stellen geeignete epoxidierte Fettstoffe dar.

Als Nucleophile, die für die Ringöffnung der Epoxidverbindungen benötigt werden, kommen bevorzugt hydroxylgruppenhaltige Verbindungen aus der Gruppe Wasser, monofunktionelle Alkohole, mehrwertige Alkohole und Carbonsäuren in Betracht.

Die monofunktionellen Alkohole enthalten 1 bis 54 Kohlenstoffatome. Typische Beispiele sind Methanol, Ethanol, Propanol-1, Propanol-2, n-Butanol, Pentanol, Hexanol, 2-Ethylhexanol, Fettalkohole mit 6 bis 22 C-Atomen, Cyclohexanol, Benzylalkohol, Octanol, Decanol, Laurylalkohol, Myristylalkohol, Cetylalkohol, Stearylalkohol, Oleylalkohol, Behenylalkohol oder Erucylalkohol oder die aus Dimer-/Trimerfettsäureestern durch Hydrierung zugänglichen Dimerdiole oder Trimerdiole. Bevorzugt ist der Einsatz von Methanol und Ethanol.

Auch die Alkoxylierungsprodukte der vorgenannten Alkohole mit bevorzugt bis zu 10 mol Alkylenoxid können für die Ringöffnung eingesetzt werden.

Die mehrwertigen Alkohole enthalten bevorzugt 2 bis 8 Hydroxylgruppen. Beispiele für mehrwertige Alkohole sind Ethylenglycol, Diethylenglycol, Polyethylenglycole im Molgewichtsbereich 300 bis 1500, 1,2-Propandiol, 1,3-Propandiol, 1,4-Butandiol, 1,6-Hexandiol, Neopentylglykol, Glycerin, Trimethylolethan, Oligoglycerine mit Kondensationsgraden von durchschnittlich 2 bis 10, Trimethylolpropan, Pentaerythrit, Sorbitol und Sorbitan.

Auch die Alkoxylierungsprodukte der vorgenannten Alkohole mit bevorzugt bis zu 10 mol Alkylenoxid pro Hydroxylgruppe können für die Ringöffnung eingesetzt werden.

Bevorzugt sind Ethylenglykol, Propan-1,2-diol, Glycerin und Trimethylolpropan.

Bevorzugte Carbonsäuren enthalten 1 bis 54 C-Atome. Beispiele für Carbonsäuren sind Ameisensäure, Essigsäure, Propionsäure, Fettsäuren mit 6 bis 22 C-Atomen, Dimer-Fettsäure, Trimerfettsäure, Ricinolsäure, Hydroxistearinsäure und Benzoesäure oder deren Mischungen.

Bevorzugt sind Mischungen von Fettsäuren mit 6 bis 18 C-Atomen, besonders bevorzugt 6 bis 12 C-Atomen.

Die Verbindungen aus der Gruppe der epoxidierten Fettstoffe können mit Verbindungen aus der Gruppe der Nucleophile zur Reaktion gebracht werden. Dabei sind erfindungsgemäß alle Kombination möglich.

Bestimmte Kombinationen von epoxidierten Fettstoffen und Nucleophilen stellen bevorzugte Ausführungsformen der Erfindung dar.

In einer Ausführungsform werden epoxidierte Triglyceride oder epoxidierte Fettsäureester monofunktioneller Alkohole mit Wasser unter Ringöffnung umgesetzt und anschließend alkoxyliert.

In einer weiteren Ausführungsform werden epoxidierte Triglyceride mit monofunktionellen Alkoholen umgesetzt und anschließend alkoxyliert.

Bevorzugte Alkohole sind Methanol und Fettalkohole mit 8 bis 18 C-Atomen.

Die Ringöffnungsreaktion epoxidierter Fettsäureester oder Triglyceride mit einem Alkohol kann gegebenenfalls von einer Umesterung mit sich selber oder anderen, nachträglich zugefügten Triglyceriden, wie zum Beispiel Palmöl, Erdnußöl, Rüböl, Baumwollsaatöl, Sojaöl, Sonnenblumenöl und Leinöl, gefolgt sein.

In einer bevorzugten Ausführungsform werden epoxidierte Triglyceride mit Carbonsäuren umgesetzt und anschließend alkoxyliert.

Geeignete Carbonsäuren sind Mischungen von Fettsäuren mit 6 bis 18 C-Atomen, besonders bevorzugt 6 bis 12 C-Atomen, sowie Ricinolsäure mit Hydroxystearinsäure.

In einer weiteren Ausführungsform werden epoxidierte Fettsäureester mit mehrwertigen Alkoholen umgesetzt und anschließend alkoxyliert. Bevorzugt sind hier die Ringöffnungs- und Umesterungsprodukte mit Alkoholen der Funktionalität 2 bis 4, insbesondere die Umsetzungsprodukte mit Ethylenglykol, Propylenglykol, oligomeren Ethylenglykolen, oligomeren Propylenglykolen, Glycerin, Trimethylolpropan oder Pentaerythrit.

Bevorzugt sind Ringöffnungsprodukte, bei denen ein molares Verhältnis zwischen epoxidiertem Fettsäureester und dem zur Umsetzung verwendetem Alkohol von 1:0,5 bis 1:10 angewandt worden ist.

In einer bevorzugten Ausführungsform werden epoxidierte Fettsäureester mit Carbonsäuren umgesetzt und anschließend alkoxyliert.

Geeignete Carbonsäuren sind Mischungen von gesättigten oder ungesättigten Fettsäuren mit 6 bis 18 C-Atomen, besonders bevorzugt 6 bis 12 C-Atomen, sowie Ricinolsäure mit Hydroxystearinsäure. Weiterhin sind Dimerfettsäure und Trimerfettsäure geeignet.

Die Epoxidverbindungen und die Nucleophile können in molaren Verhältnissen von 1 : 10 bis 10 : 1, vorzugsweise 1 : 3 bis 3 : 1 miteinander umgesetzt werden. Es ist vor allem bei epoxidierten Fettsäureestern und/oder Dicarbonsäuren mehrwertigen Alkoholen besonders bevorzugt, ein Verhältnis einzuhalten, das etwa im Bereich der Äquivalenz liegt.

Die Ringöffnungsprodukte können Hydroxylzahlen von 20 bis 400, bevorzugt 50 bis 200 aufweisen.

Die Umsetzungen epoxidierter Fettstoffe mit Nucleophilen kann bei Temperaturen zwischen 50 und 250 °C und Drücken zwischen $10^5$ und $10^6$ Pa durchgeführt werden. Ringöffnungen mit Alkoholen werden vorzugsweise in Gegenwart saurer Katalysatoren, wie Schwefelsäure und/oder p-Toluolsulfonsäure durchgeführt.

Die Ringöffnung kann in an sich bekannter Weise erfolgen, wobei es sich als vorteilhaft erwiesen hat, die Reaktion bei der Siedetemperatur des eingesetzten Nucleophils bzw. im Tem- peraturbereich von 50 bis 250 °C bei Normaldruck durchzuführen. Dabei ist es nicht zwingend erforderlich, daß die Ringöffnung vollständig abläuft. Es ist vielmehr ebenso möglich, Epoxidringöffnungsprodukte herzustellen, die noch über einen definierten Restgehalt an Epoxidsauerstoff, beispielsweise 1 bis 3 Gew.-%, verfügen. Die Reaktionszeiten liegen im allgemeinen zwischen 1 und 6 Stunden. Nach der Reaktion kann der Katalysator durch übliche Verfahren, beispielsweise durch Adsorption an einem festen Material entfernt werden oder kann nach Neutralisation mit Alkalien wie z.B. NaOH, KOH, LiOH oder $Na_2CO_3$ oder Aminen wie Ethanolamin, Morpholin oder Ammoniak im Produkt verbleiben.

Im Anschluß an die Ringöffnung der epoxidierten Fettstoffe mit einem Nucleophil erfolgt die Alkoxylierung.

Die Alkoxylierung erfolgt nach bekannten großtechnischen Verfahren mit Ethylenoxid, Propylenoxid und/oder Butylenoxid, vorzugsweise mit Ethylenoxid und/oder Propylenoxid, vorzugsweise in Gegenwart von Katalysatoren, beispielsweise Kaliumhydroxid und/oder Natriummethylat, bei Temperaturen zwischen 110 und 200 °C, vorzugsweise zwischen 140 und 175 °C und bei Drücken zwischen $10^5$ und $2 \cdot 10^6$ Pa, vorzugsweise zwischen $3 \cdot 10^5$ und $5 \cdot 10^5$ Pa. Der Alkylenoxidgehalt liegt zwischen 2 und 200 Gew.-%, vorzugsweise zwischen 20 und 100 Gew.-%, besonders bevorzugt zwischen 40 und 80 Gew.-%, jeweils bezogen auf die nichtalkoxylierten Verbindungen.

Eine besonders bevorzugte Kombination von epoxidiertem Fettstoff, Nucleophil und Alkylenoxid stellen epoxidierte Triglyceride, Fettsäuregemische aus Fettsäuren mit 6 bis 12 C-Atomen und Ethylenoxid in Mengen von 40 bis 80 Gew.% - bezogen auf die nicht alkoxylierte Verbindung - dar.

Die erfindungsgemäß zu verwendenden Alkoxylierungsprodukte sind flüssige bis hochviskose Produkte, die in Einzelfällen auch schon als fest zu bezeichnen sind. Bei Zugabe der Alkoxylierungsprodukte zu schaumbildenden Systemen tritt bereits in Gegenwart sehr geringer Mengen eine starke Minderung der Schaumbildung ein. Dabei sind die Alkoxylierungsprodukte sowohl geeignet, bereits auftretenden Schaum zu mindern als auch präventiv Schaumbildung zu vermeiden. Die Alkoxylierungsprodukte können pur oder auch in Lösung beziehungsweise Dispersion den Systemen zugesetzt werden. Sofern man Lösungen beziehungsweise Dispersionen einsetzen will, kann ein flüssiges, organisches Medium, beispielsweise ein Alkohol, Ester oder Methylenchlorid verwendet werden. Man kann aber auch Wasser als Lösemittel nehmen. Sofern die Alkoxylierungsprodukte hohe Gehalte an Ethylenoxid aufweisen, sind sie selbstemulgierbar in Wasser, das heißt ohne Zugabe von externen Emulgatoren können sie in Wasser emulgiert, beziehungsweise dispergiert werden. Sofern jedoch der hydrophobe Anteil in den Alkoxylierungsprodukten überwiegt, ist es hier notwendig, entweder andere Lösemittel zu verwenden oder externe Emulgatoren einzusetzen. Ob die Alkoxylierungsprodukte selbstemulgierbar sind oder nicht, ist durch Handversuche festzustellen, indem man die Substanzen mit Wasser versetzt und unter Rühren versucht zu emulgieren beziehungsweise dispergieren.

Die Alkoxylierungsprodukte können in der Papierindustrie bei der Zellstoffherstellung, beispielsweise bei der Sulfitzellstoffkochung, bei der Papiererzeugung und auch beim Papierstreichen (Papierbeschichten) eingesetzt werden. Bevorzugt ist der Einsatz bei der Papiererzeugung. Des weiteren sind die Alkoxylierungsprodukte verwendbar in der Nahrungsmittelindustrie, bei der Nahrungsmittelerzeugung und Verarbeitung, beispielsweise in der Zuckerindustrie bei der Rübenschwemme und beim Waschen und Schnitzeln der Zuckerrüben, bei der Extraktion des Zuckers aus den Rübenschnitzeln und der nachfolgenden Behandlung mit Kalkmilch als auch bei den mehrstufigen Verdampfern, in denen so lange Wasser entzogen wird, bis ein mit Zucker übersättigter Kristallbrei, die Füllmasse, entsteht. Ebenso können die Alkoxylierungsprodukte in der Hefeindustrie bei der technischen Herstellung von Backhefe durch Fermentation verwendet werden. Hier tritt vor allem bei der aeroben Gärungsstufe sehr viel Schaum auf, der mit den Alkoxylierungsprodukten verhindert bzw. reduziert werden kann. Des weiteren können die Alkoxylierungsprodukte auch in der Kartoffelverarbeitung eingesetzt werden, da sie mit dem schwer zu bekämpfenden Stärkeschaum fertig werden. Auch in der Lack- und Farbenindustrie können Alkoxylierungsprodukte ohne Probleme zugesetzt werden, das heißt vor allen Dingen, ohne daß sie ansonsten die Qualität der Lacke oder Farben in irgendeiner Weise beeinträchtigen und dennoch insgesamt den Schaum, der insbesondere durch Eintrag durch Luft entsteht, zerstören. Die Einsatzmenge an Alkoxylierungsprodukten variiert nach dem jeweiligen Einsatzgebiet. Prinzipiell ist man bemüht, möglichst geringe Mengen zusetzen zu müssen, alleine schon aus ökonomischen Gründen. In der Regel liegt die Einsatzmenge an den Alkoxylierungsprodukten im Bereich von 10 bis 10.000 ppm.

Die erfindungsgemäße Verwendung kann in ihrer einfachsten Form dadurch erreicht werden, daß ein oben beschriebenes Alkoxylierungsprodukt einer ohne diesen Zusatz zu unerwünscht starkem Schäumen neigenden wäßrigen System in Substanz, gelöst in einem vorzugsweise wasssermischbaren organischen Lösungsmittel oder in wäßriger Suspension beziehungsweise Dispersion zugesetzt wird. Zu den zum Schäumen neigenden wäßrigen Systemen, die mit Hilfe der erfindungsgemäß zu verwendenden Alkoxylierungsprodukte entschäumt beziehungsweise schaumreguliert werden können, gehören neben den oben genannten auch wäßrige Textilfärb- oder -vorbehandlungsbäder, Pulpen für die Zellstoff- oder Papierherstellung sowie zuckerhaltige Pflanzensäfte, wie sie in der Nahrungsmittelindustrie bei der Melasse- beziehungsweise Zuckerrübenverarbeitung anfallen.

Neben der Behandlung von Wertstoffen im Rahmen der in dieser Beschreibung bisher erwähnten Prozesse können auch solche wäßrigen Systeme erfindungsgemäß entschäumt werden, bei denen es sich um Abwässer, beispielsweise aus einem der vorstehend genannten Verfahren handelt. Die Anwendbarkeit der erfindungsgemäßen Entschäumer ist jedoch nicht auf diese Art von Abwässern limitiert, sondern jegliche Form von mit organischen, schaumbildenden Substanzen verunreinigtem Wasser kann auf diese Weise am übermäßigen Schäumen bei Agitation gehindert werden. Eine solche Agitation stellt beispielsweise die Klärung von Abwässern aus einem der o.g. Prozesse dar, aber auch die Reinigung von Abwässern aus dem kommunalen oder landwirtschaftlichen Bereich kann durch die erfindungsgemäßen Entschäumer unterstützt werden.

Es ist selbstverständlich auch möglich, die Alkoxylierungsprodukte in Abmischung mit anderen entschäumend wirkenden Verbindungen zu verwenden, z.B. mit Fettalkoholen, Ölen und Fetten, Partialglyceriden, Alkoxylierungsprodukten von Fettsäuren und Fettalkoholen, Alkoxylierungsprodukten von Polyolen und deren Partialestern, Paraffinen, feinverteilten Silicaten und Wachsen.

## Beispiele

Alle prozentualen Angaben in den Beispielen verstehen sich, sofern nicht anders vermerkt, als Gewichtsprozent.

**Beispiel 1:**

**Herstellung von ethoxyliertem, mit Carbonsäuren ringgeöffnetem Sojaölepoxid**

In einem Rührkessel wurden 126 kg (812 Mol) einer Mischung gesättigter Fettsäuren (60 Gew.-% Octansäure, 35 Gew.-% Decansäure, 3 Gew.-% Dodecansäure und 2 Gew.-% Hexansäure; SZ = 361,9, JZ <1) und 180 kg (766 Mol) epoxidiertes Sojaöl (6,8 Gew.-% Epoxid- Sauerstoff-Gehalt) vorgelegt und unter Rühren auf 170 °C erwärmt. Nachdem das Reaktionsgemisch keine Epoxidgruppen mehr enthielt (ca. 4 Stunden) wurde im Vakuum bis etwa 190 °C destilliert. Es wurde eine dunkelgelbe Flüssigkeit mit einer OHZ von 84,6, einer VZ von 239 und einer SZ von 2,4 erhalten.

423 g des Umsetzungsproduktes von Sojaölepoxid mit Carbonsäuren wurden mit 6,9 g einer 30 gew.-%igen Lösung von Kaliumhydroxid in Methanol versetzt und analog Beispiel 1 bei 140 °C mit 660 g Ethylenoxid umgesetzt. Nach Entfernen der Ethylenoxidspuren im Vakuum und Neutralisation mit Milchsäure wurde eine dunkelgelbe Flüssigkeit mit einer OHZ von 54,7 erhalten.

**Beispiel 2:**

**Herstellung von ethoxyliertem, mit Laurylalkohol ringgeöffnetem Sojaölepoxid**

In einem Rührkessel wurden 474 g Sojaölepoxid (Kenndaten wie in Beispiel 1 angegeben) und 745 g Laurylalkohol mit 3,6 g konzentrierter Schwefelsäure versetzt und auf 100 °C erwärmt. Nachdem das Reaktionsgemisch keine Epoxidgruppen mehr enthielt (ca. 3,5 Stunden) wurde mit 3,6 g Diethylethanolamin neutralisiert und der überschüssige Laurylalkohol im Vakuum bei 10 Pas und 135 °C abdestilliert. Es wurden 723 g Umsetzungsprodukt mit einer OHZ von 112, einer JZ von 20, einer VZ von 116 und einer SZ von 72 erhalten.

390 g des Umsetzungsproduktes von Sojaölepoxid mit Laurylalkohol wurden mit 4,0 g einer 30 gew.-%igen Lösung von Kaliumhydroxid in Methanol versetzt und analog Beispiel 1 bei 170 °C mit 610 g Ethylenoxid umgesetzt. Nach Entfernen der Ethylenoxidspuren im Vakuum und Neutralisation mit Milchsäure wurde eine gelbe Paste erhalten.

**Prüfung auf entschäumende Wirkung:**

Testmedium:

80    Teile atro einer Altpapier-Mischung bestehend aus:
20%   light-weight coated Papier (Kataloge)
40%   Tageszeitung
40%   Illustrierte

+ 20 Teile atro Holzschliff gebleicht auf 60° SR-Mahlgrad gemahlen

Herstellung:

Die ca. 5%ige AP-Dickstoffmischung (10 Minuten im Pulper aufgeschlagen) und der ca. 2%ige Holzschliff auf 60° SR gemahlen) werden nach Bestimmung der Stoffdichte im obigen Verhältnis gemischt und vor dem Test mit Leitungswasser von annähernd Testtemperatur auf 1% Stoffdichte verdünnt.

Test-Bedingungen:

Dieses Gemisch wird über eine Schlauchpumpe in ein Gefäß gepumpt, wobei es über eine Düse intensiv mit der eingesaugten Luft vermischt wird. Dadurch kommt es zur Schaumbildung bei gleichzeitiger Anlagerung von Luft an die im Medium vorhandenen Fasern.

Die relative Höhe des sich bildenden Schaumes wird mit Hilfe einer Skala mit mm-Einteilung abgelesen.

5kg Testmedium, 1 % Stoffdichte
Temperatur variabel zwischen Raumtemperatur und ca. 90 °C
Umpumpen mit 4 l/Minute
Lufteintrag 100 l/Stunde

Entschäumerdosierung:

Im Test werden die Entschäumer 1:10 mit Wasser vorverdünnt Dosierzeitpunkt bei Erreichen von 200 mm Schaumhöhe (ca. 1 Minute nach Versuchstart)

Dosierung in den Ansaugschlauch kurz vor der Schlauchpumpe

Die entschäumende Wirkung (in %) wird nach einer Minute (Spontan-Effekt) und nach 5 Minuten (Langzeit-Effekt) bestimmt. Sie läßt sich nach der folgenden Formel berechnen:

$$100 \text{ \%} - \left( \frac{\text{gemessener Schaumhöhe in mm}}{200\text{mm}} \times 100 \text{ \%} \right)$$

Die Ergebnisse sind in der Tabelle 1 dargestellt.

Tabelle 1:

| Entschäumerwirkung | | | | |
|---|---|---|---|---|
| Entschäumer | Menge [µl]* | T [°C] | Wirkung (nach 1 Min.) [%] | Wirkung (nach 5 Min.) [%] |
| Bsp 1 | 200 | 45 | 100 | 25 |
| Bsp 1 | 400 | 45 | 100 | 35 |
| Bsp 1 | 200 | 55 | 95 | 5 |
| Bsp 1 | 400 | 55 | 100 | 15 |
| Bsp 1 | 200 | 60 | 90 | 0 |
| Bsp 1 | 400 | 60 | 100 | 20 |
| | | | | |
| Vergl 1 | 200 | 45 | 90 | 10 |
| Vergl 1 | 200 | 55 | 70 | 0 |

Vergl. 1 ist ein Ölsäure verestertes Ethylenoxid/Propylenoxid-Blockcopolymer gemäß EP-A-264 826.

\* Die Entschäumerdosierung bezieht sich auf eine 1:10 mit Wasser vorverdünnte Lösung.

**Patentansprüche**

1. Verwendung von Alkoxylierungsprodukten von mit Nucleophilen ringgeöffneten epoxidierten Fettstoffen als Entschäumer in wäßrigen Systemen.

2. Verwendung nach Anspruch 1, dadurch gekennzeichnet, daß die Alkylenoxide Ethylenoxid und/oder Propylenoxid darstellen.

3. Verwendung nach den Ansprüchen 1 und 2, dadurch gekennzeichnet, daß die epoxidierten Fettstoffe epoxidierte Triglyceride darstellen.

4. Verwendung nach den Ansprüchen 1 bis 3, dadurch gekennzeichnet, daß die epoxidierten Fettstoffe Fettsäure-Ester monofunktioneller Alkohole darstellen.

5. Verwendung nach den Ansprüchen 1 bis 4, dadurch gekennzeichnet, daß die Nucleophile monofunktionelle Alkohole darstellen.

6. Verwendung nach den Ansprüchen 1 bis 5, dadurch gekennzeichnet, daß die Nucleophile mehrwertige Alkohole darstellen.

7. Verwendung nach den Ansprüchen 1 bis 6, dadurch gekennzeichnet, daß die Nucleophile Carbonsäuren darstellen.

8. Verwendung nach den Ansprüchen 1 bis 7, dadurch gekennzeichnet, daß das Nucleophil Wasser darstellt.

9. Verwendung nach den Ansprüchen 1 bis 8, dadurch gekennzeichnet, daß der Alkylenoxid-Gehalt zwischen 2 und 200 Gew.-%, vorzugsweise zwischen 20 und 100 Gew.-%, besonders bevorzugt zwischen 40 und 80 Gew.-%,

jeweils bezogen auf die nichtalkoxylierten Verbindungen, liegt.

10. Verwendung nach den Ansprüchen 1 bis 9, dadurch gekennzeichnet, daß der epoxidierte Fettstoff epoxidierte Triglyceride, das Nucleophil Carbonsäuren mit 6 bis 12 C-Atomen, das Alkylenoxid Ethylenoxid darstellt und der Gehalt an Ethylenoxid zwischen 40 und 80 Gew.% bezogen auf die nicht alkoxylierte Verbindung liegt.

11. Verwendung nach den Ansprüchen 1 bis 10, dadurch gekennzeichnet, daß das wäßrige System bei der Herstellung von Papier eingesetzt wird.

12. Verwendung nach den Ansprüchen 1 bis 11, dadurch gekennzeichnet, daß das wäßrige System bei der Herstellung von Lebensmitteln eingesetzt wird.

13. Verwendung nach den Ansprüchen 1 bis 12, dadurch gekennzeichnet, daß das wäßrige System bei Fermentationsprozessen auftritt.

14. Verwendung nach den Ansprüchen 1 bis 13, dadurch gekennzeichnet, daß das wäßrige System bei der Herstellung von Lacken auftritt.

15. Verwendung nach den Ansprüchen 1 bis 14, dadurch gekennzeichnet, daß das wäßrige System bei der Textilfärbung eingesetzt wird.

16. Verwendung nach den Ansprüchen 1 bis 15, dadurch gekennzeichnet, daß das wäßrige System bei der Klärung von Abwässern auftritt.

**Claims**

1. The use of alkoxylation products of epoxidized fatty compounds ring-opened with nucleophiles as defoamers in aqueous systems.

2. The use claimed in claim 1, characterized in that the alkylene oxides are ethylene oxide and/or propylene oxide.

3. The use claimed in claims 1 and 2, characterized in that the epoxidized fatty compounds are epoxidized triglycerides.

4. The use claimed in claims 1 to 3, characterized in that the epoxidized fatty compounds are fatty acid esters of monohydric alcohols.

5. The use claimed in claims 1 to 4, characterized in that the nucleophiles are monohydric alcohols.

6. The use claimed in claims 1 to 5, characterized in that the nucleophiles are polyhydric alcohols.

7. The use claimed in claims 1 to 6, characterized in that the nucleophiles are carboxylic acids.

8. The use claimed in claims 1 to 7, characterized in that the nucleophile is water.

9. The use claimed in claims 1 to 8, characterized in that the alkylene oxide content is between 2 and 200% by weight, preferably between 20 and 100% by weight and more preferably between 40 and 80% by weight, based on the non-alkoxylated compounds.

10. The use claimed in claims 1 to 9, characterized in that the epoxidized fatty compound is an epoxidized triglyceride, the nucleophile is a carboxylic acid containing 6 to 12 carbon atoms, the alkylene oxide is ethylene oxide and the ethylene oxide content is between 40 and 80% by weight, based on the non-alkoxylated compound.

11. The use claimed in claims 1 to 10, characterized in that the water-containing system is used in the manufacture of paper.

12. The use claimed in claims 1 to 11, characterized in that the water-containing system is used in the manufacture of foods.

13. The use claimed in claims 1 to 12, characterized in that the water-containing system occurs in fermentation processes.

14. The use claimed in claims 1 to 13, characterized in that the water-containing system occurs in the production of lacquers.

15. The use claimed in claims 1 to 14, characterized in that the water-containing system is used in the dyeing of textiles.

16. The use claimed in claims 1 to 15, characterized in that the water-containing system occurs in the treatment of wastewaters.

**Revendications**

1. Utilisation de produits d'alcoxylation de matières grasses époxydées dont le cycle a été ouvert avec des agents nucléophiles, en tant qu'agent antimousse dans des systèmes aqueux.

2. Utilisation selon la revendication 1,
caractérisée en ce que
les oxydes d'alkylène signifient l'oxyde d'éthylène et/ou l'oxyde de propylène.

3. Utilisation selon les revendications 1 et 2,
caractérisée en ce que
les matières grasses époxydées signifient des triglycérides époxydés.

4. Utilisation selon les revendications 1 à 3,
caractérisée en ce que
les matières grasses époxydées signifient des esters d'acide gras et d'alcools monofonctionnels.

5. Utilisation selon les revendications 1 à 4,
caractérisée en ce que
les agents nucléophiles signifient des alcools monofonctionnels.

6. Utilisation selon les revendications 1 à 5,
caractérisée en ce que
les agents nucléophiles signifient des alcools plurifonctionnels.

7. Utilisation selon les revendications 1 à 6,
caractérisée en ce que
les agents nucléophiles signifient des acides carboxyliques.

8. Utilisation selon les revendications 1 à 7,
caractérisée en ce que
l'agent nucléophile signifie l'eau.

9. Utilisation selon les revendications 1 à 8,
caractérisée en ce que
la teneur en oxyde d'alkylène se situe entre 2 et 200 % en poids, de préférence entre 20 et 100 % en poids et d'une manière particulièrement préférée entre 40 et 80 % en poids, à chaque fois rapporté aux composés non alcoxylés.

10. Utilisation selon les revendications 1 à 9,
caractérisée en ce que
la matière grasse époxydée représente des triglycérides époxydés, l'agent nucléophile des acides carboxyliques ayant de 6 à 12 atomes de carbone, l'oxyde d'alkylène, de l'oxyde d'éthylène et en ce que la teneur en oxyde se situe entre 40 et 80 % en poids rapporté au composé non alcoxylé.

**11.** Utilisation selon les revendications 1 à 10,
caractérisée en ce que
le système aqueux est mis en oeuvre pour la fabrication du papier.

**12.** Utilisation selon les revendications 1 à 11,
caractérisée en ce que
le système aqueux est mis en oeuvre pour la fabrication de produits alimentaires.

**13.** Utilisation selon les revendications 1 à 12,
caractérisée en ce que
le système aqueux survient dans les processus de fermentation.

**14.** Utilisation selon les revendications 1 à 13,
caractérisée en ce que
le système aqueux survient au cours de la production de laques.

**15.** Utilisation selon les revendications 1 à 14,
caractérisée en ce que
le système aqueux est mis en oeuvre pour la teinture des textiles.

**16.** Utilisation selon les revendications 1 à 15,
caractérisée en ce que
le système aqueux survient au cours de l'épuration des eaux usées.